Europäisches Patentamt

(19) European Patent Office

Office européen des brevets

(11) Veröffentlichungsnummer: **0 101 047**
**B1**

(12) **EUROPÄISCHE PATENTSCHRIFT**

(45) Veröffentlichungstag der Patentschrift:
19.11.87

(21) Anmeldenummer: 83107828.2

(22) Anmeldetag: 09.08.83

(51) Int. Cl.⁴: **C 07 C 17/42,** C 07 C 17/38,
C 07 C 21/12, C 07 C 21/10,
H 01 B 3/24, H 01 F 27/14

(54) Verfahren zur Stabilisierung von Chlorkohlenwasserstoffen, verfahrensgemäss stabilisierte Chlorkohlenwasserstoff und ihre Verwendung.

(30) Priorität: 12.08.82 DE 3230048

(43) Veröffentlichungstag der Anmeldung:
22.02.84 Patentblatt 84/8

(45) Bekanntmachung des Hinweises auf die Patenterteilung:
19.11.87 Patentblatt 87/47

(84) Benannte Vertragsstaaten:
AT BE CH DE FR GB IT LI NL SE

(56) Entgegenhaltungen:
EP - A - 0 023 540
EP - A - 0 033 068
DD - A - 118 193
DE - A - 3 007 633
DE - B - 1 493 354
GB - A - 1 287 161
US - A - 3 751 494
US - A - 4 312 794

PATENTS ABSTRACTS OF JAPAN, unexamined
application, Section C, Band 1, Nr. 131, 28. Oktober
1977, THE PATENT OFFICE JAPANESE GOVERNMENT,
Seite 3 259 C 77

Die Akte enthält technische Angaben, die nach dem
Eingang der Anmeldung eingereicht wurden und die
nicht in dieser Patentschrift enthalten sind.

(73) Patentinhaber: WACKER-CHEMIE GMBH,
Prinzregentenstrasse 22, D-8000 München 22 (DE)

(72) Erfinder: Blum, Klaus, Dr. Dipl.-Chem., Amatiweg 5,
D-8263 Burghausen (DE)
Erfinder: Strasser, Rudolf, Dr. Dipl.-Chem., Lindacher
Strasse 58, D-8263 Burghausen (DE)

## Beschreibung

Die Erfindung betrifft ein Verfahren zur Stabilisierung von Chlorkohlenwasserstoffen, verfahrensgemäss stabilisierte Chlorkohlenwasserstofe sowie ihre Verwendung als dielektrische Flüssigkeit.

Chlorkohlenwasserstoffe sind technisch nur mit stabilisierenden Zusätzen handhabbar. Üblicherweise handelt es sich bei den genannten Zusätzen um im zu stabilisierenden Chlorkohlenwasserstoff lösliche Substanzen, die als Säurefänger oder als Antioxidantien wirken. In der Praxis werden als Säurefänger vielfach Epoxide eingesetzt. Nachteilig an diesen Epoxiden ist ihre Toxizität: so wird bei Epoxiden mit Oxiran-Struktur ein mutagenes und/oder karzinogenes Potential vermutet.

In der EP-A-33 068 wird ein Verfahren zur Reduzierung des Chloridgehaltes von mit Wasser und HCl verunreinigten ungesättigten Chlorkohlenwasserstoffen beschrieben, in dem HCl und Wasser durch Zugabe von Amin und Molekularsieb abgefangen werden.

Gemäss DD-A-118 193 kann Wasser mit Hilfe von Zeolith aus Transformatorenflüssigkeiten entfernt werden.

Die EP-A-25 540 lehrt die stabilisierende Wirkung einer Auswahl tertiärer Amine auf Halogenkohlenwasserstoffe.

Es ist ferner aus DE-OS 2 833 586 bereits bekannt, Magnesiumoxid als Stabilisierungsmittel für Chlorkohlenwasserstoffe einzusetzen.

Aufgabe der Erfindung war es, die Epoxide zumindest teilweise durch ungiftige Substanzen zu ersetzen. Insbesondere war es Aufgabe der Erfindung, stabilisierende Zusätze für Chlorkohlenwasserstoffe zu finden, die eingeschleppte und/oder entstehende Säure und Metallsalze im Chlorkohlenwasserstoff abfangen und die über lange Zeiträume wirksam sind, was insbesondere für den Einsatz z. B. von Tetrachlorethen als Transformatorenflüssigkeit gefordert wird.

Es wurde nun gefunden, dass diese Aufgabe gelöst werden kann durch ein Verfahren zum Stabilisieren von Chlorkohlenwasserstoffen, vorzugsweise Trichlorethen oder Tetrachlorethen, die lösliche Stabilisatoren aus der Gruppe der Amine, der Phenole, der Alkene, der Ester, der Nitroverbindungen, Ethanol und der Epoxide enthalten, das dadurch gekennzeichnet ist, dass die Chlorkohlenwasserstoffe mit 0,001 bis 5 Gewichtsprozenten, bezogen auf das Gewicht des zu stabilisierenden Chlorkohlenwasserstoffs, an Zeolith und 5 bis 25 Gewichtsprozent, bezogen auf die Menge vorhandenen Zeoliths, mit Zinkoxyd und/oder Natriumcarbonat in Kontakt gebracht werden.

Im Rahmen der Erfindung können natürlich vorkommende oder künstlich hergestellte (synthetische) Zeolithe eingesetzt werden. Es handelt sich hierbei um kristalline Alumosilikate, die durch die allgemeine Formel

$$Me_{2/z}O \cdot Al_2O_3 \cdot xSiO_2 \cdot yH_2O$$

charakterisiert werden können.

Me steht für Wasserstoff, Ammonium und ein- oder mehrwertige Metallkationen. Beispiele für ein- und mehrwertige Metallkationen sind Alkalimetallkationen, insbesondere Na- und K-Kationen, Erdalkalimetallkationen, wie Mg-, Ca-Kationen, Seltenerdmetallkationen, wie La-, Ce, Übergangsmetallkationen, wie Ni, ferner Zink-, Eisen-, Cobalt-, Mangan-Kationen und andere.

z ist die Wertigkeit von Me und nimmt die Werte 1, 2 und 3 an, insbesondere 1 und 2.

x nimmt Werte von 1,8 – 12 an; y steht für 0 bis ca. 8.

Beispiele für erfindungsgemäss einzusetzende Zeolithe sind Faserzeolithe, wie Mordenit, Sodalith, Natrolith, Thomsonit, Blätter-Zeolithe, wie Phillipsit, Heulandit, Stilbit, Würfel-Zeolithe, wie Faujasit, Gmelinit und Chabasit, synthetische Zeolithe, wie Zeolith X, Y, A, L, T.

Die erfindungsgemäss einzusetzenden Zeolithe weisen Porendurchmesser von 2 bis 10 A auf. Das Schüttgewicht liegt in der Regel im Bereich von 600–800 g/dm$^3$ bei Formkörpern und 300–400 g/dm$^3$ bei pulverförmigen Zeolithen.

Die Zeolithe werden in Pulverform oder als Formkörper eingesetzt. Die Pulver weisen zumeist Teilchengrössen von 1 bis 10 μ auf. Die Formkörper werden aus den Pulvern gewonnen und können Bindemittel enthalten, wie Wasserglas, Kieselsäureester, Natriumcarbonat sowie andere Metalloxide und/oder -carbonate. Beispiele für Formkörper sind Kugeln, Würfel, Zylinder, Hohlstränge und dergleichen.

Beispiele für erfindungsgemäss zu stabilisierende Chlorkohlenwasserstoffe sind Methylenchlorid, 1.1.1-Trichlorethan und olefinisch ungesättigte Chlorkohlenwasserstoffe, wie Trichlorethen, Tetrachlorethen und Hexachlorbutadien. Bevorzugt sind Trichlorethen und Tetrachlorethen.

Das erfindungsgemässe «In-Kontakt-Bringen» kann in mannigfacher Weise erfolgen und richtet sich nach der Verwendung, der Art der Lagerhaltung usw. des zu stabilisierenden Chlorkohlenwasserstoffs.

Im einfachen Fall wird Zeolith als Pulver oder als Formkörper lose zugegeben, so dass der Zeolith in Suspension und/oder als Bodenkörper im Chlorkohlenwasserstoff vorliegt. Oftmals muss jedoch im zu stabilisierenden Chlorkohlenwasserstoff auf Ausschluss von Feststoffen, insbesondere Stäuben, Wert gelegt werden. In solchen Fällen kommen die erfindungsgemässen Zeolithe mit Umhüllungen versehen zum Einsatz, die den Feststoff zurückhalten, aber für den Chlorkohlenwasserstoff durchlässig sind. Solche Umhüllungen können Filterkerzen, Filtertaschen, Membranfilter, Geflechte und dergleichen sein. Spezielle Beispiele für Umhüllungsmaterialien sind Glasfaser, Polyamid-, Zelluloseacetat-, Zellulosenitrat-, Polytetrafluorethylen-, Polyvinylidenfluorid-, Polyestergewebe, Metalldrahtgewebe und Mischfasergewebe, wie beispielsweise ein Gewebe aus Hanf, Zellulosepulp und Polyvinylchlorid-Polyvinylacetat-Mischpolymerisat.

Das erfindungsgemässe «In Kontakt-Bringen» der umhüllten Zeolithe kann z.B. durch einfaches Eintauchen in den Chlorkohlenwasserstoff erfolgen. Es können jedoch auch, je nach praktischen Gegebenheiten, kompliziertere Vorrichtungen vorgesehen sein: beispielsweise können Teilströme des Chlorkohlenwasserstoffs durch Filteranlagen, die erfindungsgemäss einzusetzenden Zeolith enthalten, gepumpt werden. Daneben kommen Anordnungen in Betracht, wie sie beispielsweise zur Feststoffextraktion bekannt sind.

Die Mengen an erfindungsgemäss einzusetzendem Zeolith richten sich nach der Belastung des Chlorkohlenwasserstoffs. Die Mengen liegen im Bereich von 0,001 Gew.-% bis 5 Gew.-%, bezogen auf die Menge des zu stabilisierenden Chlorkohlenwasserstoffs. Für die meisten Zwecke reichen Mengen von 0,002 bis 0,1 Gew.-%, bezogen auf die Menge des zu stabilisierenden Chlorkohlenwasserstoffs aus.

Erfindungsgemäss kann in dem Temperaturbereich stabilisiert werden, in dem der Chlorkohlenwasserstoff handhabbar ist. Zumeist liegen die Temperaturen im Bereich der Temperatur der umgebenden Atmosphäre bis zum Siedepunkt des zu stabilisierenden Chlorkohlenwasserstoffs.

Der erfindungsgemässe Begriff des «Stabilisierens» soll sowohl das Stabilisieren unverbrauchten Chlorkohlenwasserstoffs umfassen, wie auch das Nachstabilisieren bzw. Regenerieren ge- bzw. verbrauchter Chlorkohlenwasserstoffe.

Typische Beispiele für erfindungsgemäss stabilisierte Chlorkohlenwasserstoffe sind Tetrachlorethen und Trichlorethen, jeweils enthaltend:

10–50 Gew.-ppm eines Amins, dessen Siedepunkt bei 1 bar zwischen 50 und 150 °C liegt (z.B. Triethylamin, Diisopropylamin, Dimethylisobutylamin, insbesondere Diisopropylamin)

5–500 Gew.-ppm Antioxidantien (wie z.B. N-Methylpyrrol, N-Methylmorpholin, Alkylphenole, die mit 1–3, ggf. verzweigten Alkylketten in o- und/oder p-Stellung substituiert sind) und/oder

500–3000 ppm substituierte Alkene (wie Diisobutylen, Amylen und Cycloheptatrien) und

10–1000 Gew.-ppm Zeolith sowie 0–250 ppm fester Co-Stabilisator.

Beispiele für die obengenannten Alkylphenole sind p-Kresol, o-Kresol, 2.6-Dimethylphenol, 2.4.6-Trimethylphenol, p-Isopropylphenol, p-tert.-Butylphenol, 2.6-Di-tert.-Butylphenol, p-Amylphenol und andere.

Erfindungsgemäss hochstabilisierte Chlorkohlenwasserstoffe, insbesondere solche Tetrachlorethylen- und Trichlorethylen-Typen, die gegen den zersetzenden Einfluss von metallischem Aluminium stabilisiert sind, können weiterhin Metalldesaktivatoren in Mengen von 500 bis 4000 ppm enthalten, wie beispielsweise Ester (z.B. Ethylacetat), cyclische Ether (z.B. Tetrahydrofuran) und cyclische Amine.

Wenn es auch Aufgabe der Erfindung war, die toxischen Epoxide möglichst vollständig zu ersetzen, so können die erfindungsgemäss stabilisierten Chlorkohlenwasserstoffe, insbesondere erfindungsgemäss stabilisiertes Trichlorethylen und Tetrachlorethylen auch Epoxide enthalten, umso mehr, als sich das Verfahren des erfindungsgemässen Stabilisierens auch auf verbrauchte Chlorkohlenwasserstoffe bezieht. Ferner werden die Epoxide in der Praxis nicht nur als Stabilisatoren eingesetzt, sondern dienen daneben auch als Indikatoren für den effektiven Stabilisierungszustand des Chlorkohlenwasserstoffs. Die Menge an Epoxid beträgt dabei 50 Gew.-ppm bis 7000 Gew.-ppm. 50 bis 100 Gew.-ppm Epoxid reichen für die Indikatorfunktion aus. Gleichwohl sei festgestellt, dass erfindungsgemäss stabilisierte Chlorkohlenwasserstoffe auch ohne Zusatz von Epoxiden hohen Anforderungen an die Belastbarkeit des Chlorkohlenwasserstoffs erfüllen.

Zur Prüfung auf Stabilität wurden die erfindungsgemäss stabilisierten Chlorkohlenwasserstoffe dem folgenden Test unterworfen:

Es wurde der beschleunigte Oxidationstest nach MIL-T 81 533 A oder analoge Test in Gegenwart von Stahl durchgeführt. Als Testapparatur diente ein mit 200 ml erfindungsgemäss stabilisierten Chlorkohlenwasserstoffs gefülltes Siedegefäss mit Rückflusskühler, das mittels einer 150 Watt Glühlampe beheizt wurde. In die Testflüssigkeit wurde ein stetiger wasserdampfgesättigter Sauerstoffstrom (10 bis 12 Blasen pro Minute) geleitet. Weiter war ein Streifen aus metallischem Aluminium (bzw. Stahl) so angeordnet, dass er zur Hälfte in die Testflüssigkeit eintauchte. Ein zweiter Aluminiumstreifen (Stahlstreifen) befand sich am Gefässboden.

Als Stabilitätskriterium wurde die Zeit herangezogen, die der stabilisierte Chlorkohlenwasserstoff den obigen Testbedingungen ausgesetzt werden musste, bis ein HCl-Gehalt von 0,02 Gew.-% erreicht war. Der HCl-Gehalt wurde durch regelmässige Probeentnahme und anschliessende Titration mit 0,1 nNaOH gegen Phenolphthalein ermittelt.

Die erfindungsgemäss stabilisierten Chlorkohlenwasserstoffe, insbesondere erfindungsgemäss stabilisiertes Trichlorethylen und Tetrachlorethylen können Verwendungszwecken zugeführt werden, die auch bereits bisher für stabilisierte Chlorkohlenwasserstoffe bekannt waren. Es sind dies z.B. die Verwendung zur Metallentfettung, die Verwendung als Lacklösemittel, die Verwendung als Trockenreinigungsmittel und andere.

Für erfindungsgemäss mit Zeolith stabilisiertes Tetrachlorethylen, das neben den bereits beschriebenen anderen stabilisierenden Zusätzen noch Farbstoffe, Penetrieröle und dergleichen enthalten kann, wurde weiterhin die spezielle Anwendung als dielektrische Flüssigkeit in elektrischen Geräten, insbesondere in Transformatoren, gefunden.

Die erfindungsgemäss eingesetzten Zeolithe fangen entstehende Säure und Metallsalze sofort ab und schalten damit von vornehrein eine wesentliche Ursache für die Zersetzung der Chlorkohlenwasserstoffe aus. Darüberhinaus bleibt die erfindungsgemässe Stabilisierung über sehr lange Zeiträume wirksam, wie dies insbesondere für den oben beschriebenen speziellen Anwendungszweck (erfindungsgemäss stabilisiertes Tetrachlorethen als Transformatorenflüssigkeit) gefordert wird.

Die Erfindung wird nun anhand von Beispielen näher erläutert:

Beispiel 1

Proben von je 500 ml Tetrachlorethen, stabilisiert mit 50 ppm N-Methylmorpholin, 50 ppm 2.4- Di-tert.-Butylphenol, 10 g Zeolith A in der K-Form (Kugeln vom Durchmesser 1–3 mm) sowie 2,5 g eines festen Co-Stabilisators der in der nachfolgenden Tabelle 1 beschriebenen Art (Tablettenform vom Durchmesser 3 mm), wurden dem beschleunigten Oxidationstest unterworfen.

Ergebnisse:

Tabelle 1

| Co-Stabilisator | pH-Wert | nach h | Standzeit bis zum Erreichen der Aciditätsgrenze |
|---|---|---|---|
| Na₂Co₃ | 8,3 | 350 | >500 |
| ZnO | 8,2 | 200 | >500 |

Der beschleunigte Oxidationstest wurde nach 500 Stunden abgebrochen, ohne dass die Aciditätsgrenze von 0,02 Gew.-% HCl erreicht wurde.

Vergleichsversuch 1

Proben von je 500 ml Methylenchlorid, stabilisiert mit 3,2 Gew.-% Ethanol und 10 g Zeolith A in der K-Form wurden dem beschleunigten Oxidationstest in Gegenwart von Stahl unterworfen.

Nach 550 Stunden lag der pH-Wert bei 6.8.

Beispiel 2

Es wurde die Arbeitsweise gemäss Vergleichsversuch 1 wiederholt, mit der Abänderung, dass zusätzlich 2 g Zinkoxid zugesetzt wurden.

Nach 550 Stunden lag der pH-Wert bei 7.3.

Vergleichsversuch 2

Je 500 ml Trichlorethen, stabilisiert mit 50 ppm 4-tert.-Butylphenol, 20 ppm N-Methylpyrrol, 20 ppm Diisopropylamin und 5 g eines Zeoliths der in der nachstehenden Tabelle 2 bezeichneten Art wurden in Gegenwart von Stahl dem beschleunigten Oxidationstest unterzogen.

Ergebnisse:

Tabelle 2

| Zeolith | | pH-Wert | nach h | Standzeit bis zum Erreichen der Aciditätsgrenze |
|---|---|---|---|---|
| Zeolith A K-Form | Kugeln 1-3 mm | 9,5 | 48 | >500 |
| Zeolith A K-Form | Kugeln 3-6 mm | 9,1 | 48 | >500 |
| Zeolith A Na-Form | Kugeln 3-6 mm | 7,3 | 500 | >500 |
| Zeolith A Na-, K-Form | Stäbchen 6 mm | 8,9 | 186 | >500 |

Das molare Verhältnis von Na : K im Zeolith A in der Na-, K-Form betrug 3 : 1.

Beispiel 3

Es wurde die Arbeitsweise gemäss Vergleichsversuch 2 wiederholt mit 3 g Zeolith A in der K-Form sowie zusätzlich 0,75 g eines festen Co-Stabilisators in Tablettenform der in Tabelle 3 angegebenen Art.

Ergebnisse:

Tabelle 3

| Co-Stabilisator | pH-Wert | nach h | Standzeit bis zum Erreichen der Aciditätsgrenze |
|---|---|---|---|
| Na₂CO₃ | 10,3 | 350 | >500 |
| ZnO | 7,3 | 170 | >500 |

Der Oxidationstest wurde nach 500 Stunden abgebrochen, ohne dass die Aciditätsgrenze erreicht war.

**Patentansprüche**

1. Verfahren zum Stabilisieren von Chlorkohlenwasserstoffen, die lösliche Stabilisatoren aus der Gruppe der Amine, der Phenole, der Alkene, der Ester, der Nitroverbindungen, Ethanol und der Epoxide enthalten, dadurch gekennzeichnet, dass die Chlorkohlenwasserstoffe mit 0,001 bis 5 Gewichtsprozenten, bezogen auf das Gewicht des zu stabilisierenden Chlorkohlenwasserstoffs, an Zeolith und 5 bis 25 Gewichtsprozent, bezogen auf die Menge vorhandenen Zeoliths, mit Zinkoxyd und/oder Natriumcarbonat in Kontakt gebracht werden.

2. Verfahren nach Anspruch 1, dadurch gekennzeichnet, dass als Chlorkohlenwasserstoffe Trichlorethen oder Tetrachlorethen eingesetzt werden.

3. Verwendung von Tetrachlorethen nach Anspruch 2 als dielektrische Flüssigkeit in Transformatoren.

**Claims**

1. Process for the stabilisation of chlorinated hydrocarbons which contain soluble stabilisers from the group comprising amines, phenols, alkenes, esters, nitro compounds, ethanol and epoxides, characterised in that the chlorinated hydrocarbons are brought into contact with from 0.001 to 5% by weight, based on the weight of the chlorinated hydrocarbon to be stabilised, of zeolite and from 5 to 25% by weight, based on the amount of zeolite, of zinc oxide and/or sodium carbonate.

2. Process according to claim 1, characterised in that trichloroethylene or tetrachloroethylene are used as chlorinated hydrocarbons.

3. Use of tetrachloroethylene according to claim 2 as dieletric fluid in transformers.

**Revendications**

1. Procédé pour stabiliser des hydrocarbures chlorés contenant des stabilisants solubles appartenant à l'ensemble constitué par les amines, les phénols, les alcènes, les esters, les composés nitrés, l'éthanol et les époxydes, procédé caractérisé en ce qu'on met les hydrocarbures chlorés en contact avec de 0,001 à 5% en poids d'une zéolite, par rapport au poids de l'hydrocarbure chloré à stabiliser, et avec de 5 à 25% en poids, par rapport à la quantité de la zéolite présente, d'oxyde de zinc et/ou de carbonate de sodium.

2. Procédé selon la revendication 1 caractérisé en ce que l'hydrocarbure chloré utilisé est le trichloréthène ou le tétrachloréthène.

3. Application du tétrachloréthéne selon la revendication 2 comme liquide diélectrique dans des transformateurs.